# EUROPEAN PATENT APPLICATION

(11) **EP 2 199 281 A1**
(43) Date of publication of application: **23.06.2010**
(21) Application number: 08022166.6
(22) Date of filing: 19.12.2008
(51) Int. Cl.: C07C 327/06, C07C 327/22, C08G 75/26, A61K 9/50

(54) **Thioic acids as building block for polythioesters**

(71) Applicant: DSM IP Assets B.V., 6411 TE Heerlen (NL)
(72) Inventor: Boerakker, Mark, Johannes, 5654 NS Eindhoven (NL); Dias, Aylvin, Jorge, Angelo, Athanasius, 6213 HE Maastricht (NL)
(74) Representative: Vandevijver, Pascale

(57) **Abstract**

The present invention relates to thioic acids according to formula I, suitable as building block for polythioesters; 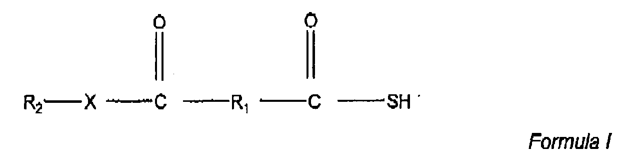 Wherein X is O, N or S and whereby
R1 is chosen from (-CH₂-)₂, -CH₂-O-CH₂- or (-CH₂-)₃ ;
if R1=(-CH2-)2 and X=O, R2 is chosen from a non-degradable polymer with a Mw>6000, a degradable polymer or oligomer, an aliphatic compound comprising at least 2 C atoms and a Mw<5000 or an aromatic compound comprising at least 2 aromatic rings;
if R1=(-CH2-)2 and X=N or S or if R1=(-CH2-)3, R2 is chosen from a monomer, oligomer or polymer.
If R1 = (-CH2-)2, R2 is preferably chosen from polyethyleneglycol (PEG) with a Mw>6000 or a degradable polymer or oligomer.
If R1=(-CH2-)2 and X=N or S or if R1=(-CH2-)3, R2 is preferably chosen from a biodegradable oligomer or polymer chosen from poly (lactide) (PLLA), polyglycolide (PGA), co-oligomers or copolymers of PLA and PGA (PLGA), poly(anhydrides), poly(trimethylenecarbonates), poly(orthoesters), poly(dioxanones), poly(ε-caprolactones) (PCL) or polyesteramides.

## Description

The present invention relates to new thioic acids as building blocks for polythioesters. The present invention further relates to a process for the preparation of thioic acids. The present invention also relates to polythioesters prepared from the thioic acids. The present invention further relates to medical devices comprising the polythioesters.

More in particular the invention relates to new thioic acids to produce polymers such as block copolymers or cross-linked networks that are suitable for medical applications. The invention provides polymers that may degrade completely when used in the human or animal body, minimizing residual components that may be toxic or otherwise undesirable.

Thioic acids are known in the prior art. From for example WO-A-2007028612 it is known that thioic acids can be used as building blocks for polythioesters. However the thioic acid containing compounds described so far are thioic acid compounds which comprise hydrophobic parts.

The polythioesters made from the thioic acid compounds comprising hydrophobic parts render degradation products with limited water solubility. The starting thioic acids will highly influence the polythioester properties, such as degradation or biodegradation but also the physical properties, the drug solubility and the release from polythioesters. For in vivo setting systems one needs to use water-soluble thioic acids which are not available at present. Finally, the hydrophobic nature limits their use for preparing hydrogel materials for biomedical applications.

The object of the present invention is to overcome the above mentioned disadvantages by providing a range of new thioic acids.

A still further object of the present invention is to provide the production of polythioesters from the new thioic acids.

It is still a further object of the present invention to provide polythioesters which hydrophilicity/hydrophobicity properties that can be tuned more easily. The ability to tune these properties would be of a great advantage in selecting a drug which might be incorporated into the polythioester. In this way the polythioesters can be tailored to reach optimal drug/polymer affinity and to obtain a desired release time.

The object of the present invention is achieved in providing new thioic acids according to formula I, suitable as building block for polythioesters: 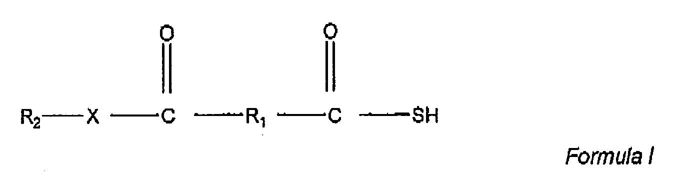 Wherein X is O, N or S and whereby
R1 is chosen from (-CH₂-)₂, -CH₂-O-CH₂- or (-CH₂-)₃;
R2 is chosen from a low molecular weight compound having a Mw<1000g/mol, oligomer or polymer with the proviso that
-if R1=(-CH₂-)₂ and X=O, R2 is chosen from non-degradable polymer with a Mw>6000, a degradable oligomer or polymer or an aliphatic compound comprising at least 2 C atoms and a Mw<5000.

Surprisingly it has been found that with the availability of the new thioic acids the hydrophilicity/ hydrophobicity balance of the resulting polythioesters can be better tuned which results in better options to tune drug compatibility with the matrix. In this way release characteristics, physical properties and degradation behaviour can be influenced. A further advantage is that the new thioic acids enable the preparation of waterborne formulations because of their more hydrophilic structure.

Finally the new thioic acids allow the preparation of hydrogels.

Examples of low molecular weight compounds having a Mw<1000g/mol are aliphatic compounds chosen from ethane, propane, butane, pentane, ethyleneglycol, diethyleneglycol, triethyleneglycol,

Examples of oligomers or polymers are poly (vinyl alcohol) (PVA), poly (ethylene oxide), poly (ethylene oxide)-co-poly(propylene oxide) block co-oligomers or copolymers (poloxamers, meroxapols), poloxamines, poly(urethanes), polyesteramides, polythioesteramides, poly((polyethyleneoxide)-co-poly(butyleneterephtalate)), poly (vinyl pyrrolidone), poly (ethyl oxazoline), carboxymethyl cellulose, hydroxyalkylated celluloses such as hydroxyethyl cellulose and methylhydroxypropyl cellulose, poly(L-lactide) (PLLA), poly (DL-lactide) (PDLLA), polyglycolide (PLGA), co-oligomers or copolymers of lactic acid and glycolic acid (PLGA), poly(anhydrides), poly(trimethylenecarbonates), poly(orthoesters), poly(dioxanones), poly(ε-caprolactones) (PCL), polyanhydrides and copolymers or co-oligomers or blends thereof.

Examples of non-degradable polymers with a Mw>6000 are poly (vinyl alcohol) (PVA), poly (ethylene oxide), poly (ethylene oxide)-co-poly(propylene oxide) block co-oligomers or copolymers (poloxamers, meroxapols), poloxamines, poly(urethanes), poly((polyethyleneoxide)-co-poly(butyleneterephtalate)), poly (vinyl pyrrolidone), poly (ethyl oxazoline), carboxymethyl cellulose, hydroxyalkylated celluloses such as hydroxyethyl cellulose and methylhydroxypropyl cellulose.

Examples of degradable or biodegradable oligomers or polymers are poly(L-lactide) (PLLA), poly (DL-lactide) (PDLLA), polyglycolide (PLGA), co-oligomers or copolymers of lactic acid and glycolic acid (PLGA), poly(anhydrides), poly(trimethylenecarbonates), poly(orthoesters), poly(dioxanones), poly(ε-caprolactones) (PCL), polyanhydrides and copolymers or co-oligomers or blends of any of the above-mentioned polymers. In particularly preferred degradable oligomers or polymers are PLLA, PLGA, PCL and copolymers of PCL and lactide or glycolide, poly(urethanes), poly (hydroxy acids), polycarbonates, polyaminocarbonates, polyphosphazenes, poly(propylene)fumarates, polyesteramides, polythioesteramides, polyoxaesters, poly(maleic acids), polyacetals, polyketals or natural polymers such as starch, polypeptides, polyhydroxyalkanoates, fibrin, chitin, chitosan, polysaccharides or carbohydrates such as polysucrose, hyaluronic acid, dextran and similar derivatives thereof, heparan sulfate, chondroitin sulfate, heparin, or alginate, and proteins such as gelatin, collagen, albumin, or ovalbumin, or co-oligomers or copolymers, or blends thereof.

If R1=(-CH₂-)₂ and X=O, R2 may also be chosen from aliphatic compounds comprising at least 2 C atoms and a Mw<5000 such as ethane, propane, butane, higher alkanes such as decane, dodecane, diethyleneglycol, triethyleneglycol.

As used herein, the term "degradable" refers to a material having a molecular structure, which can decompose to smaller molecules. Such degradation or decomposition can be by various chemical mechanisms. For example, a degradable polymer can be hydrolytically degradable in which water reacts with the polymer to form two or more molecules from the polymer by chemical bonds in the molecule being hydrolyzed, thus producing smaller molecules. Many so-called degradable polymers are not completely degradable and have to be excreted, which puts strain on the kidneys and the renal system.

As used herein the term "biodegradable" refers to oligomers or polymers are materials that experience (accelerated) degradation by the action of biological agents produced by living organisms. Organic material can be degraded aerobically with oxygen or an-aerobically without oxygen.

As used herein, the term oligomer means a molecule of intermediate relative molecular mass, the structure of which essentially comprises a small plurality of units derived, actually or conceptually, from molecules of lower relative molecular mass. It is to be noted that a molecule is regarded as having an intermediate relative molecular mass if it has properties which do vary significantly with the removal of one or a few of the units. It is also to be noted that, if a part or the whole of the molecule has an intermediate relative molecular mass and essentially comprises a small plurality of units derived, actually or conceptually, from molecules of lower relative molecular mass, it may be described as oligomer, or by oligomeric used adjectivally. In general, oligomers have a molecular weight of more than 200 Da, such as more than 400, 800, 1000, 1200, 1500, 2000, 3000, 4000, or more than 8000 Da.

As used herein, the term polymer means a molecule of high relative molecular mass, the structure of which essentially comprises the multiple repetitions of units derived, actually or conceptually, from molecules of low relative molecular mass. Such polymers may include crosslinked networks, branched polymers and linear polymers. It is to be noted that in many cases, especially for synthetic polymers, a molecule can be regarded as having a high relative molecular mass if the addition or removal of one or a few of the units has a negligible effect on the molecular properties. This statement fails in the case of certain macromolecules for which the properties may be critically dependent on fine details of the molecular structure. It is also to be noted that, if a part or the whole of the molecule has a high relative molecular mass and essentially comprises the multiple repetition of units derived, actually or conceptually, from molecules of low relative molecular mass, it may be described as either polymer or by macromolecular or polymeric used adjectivally. In general, polymers have a molecular weight of more than 8000 Da, such as more than 10.000, 12.000, 15.000, 25.000, 40.000, 100.000 or more than 1.000.000 Da.

The present invention further relates to new thioic acids according to formula II, suitable as building block for polythioesters, 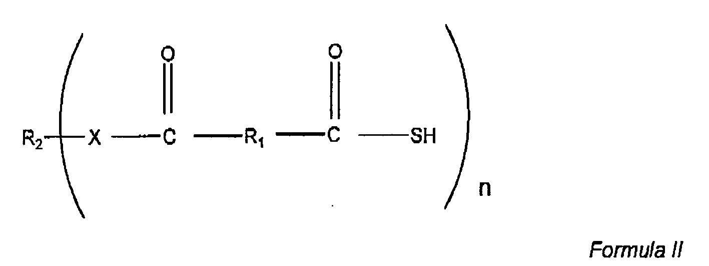 Wherein X is O, N or S and n >1;
R1 is chosen from (-CH2-)₂, -CH₂-O-CH₂- or (-CH₂-)₃ and
R2 is chosen from a low molecular weight compound having a Mw<1000 Da, oligomer or polymer.

Preferably R2 is chosen from a degradable oligomer or polymer. More preferably R2 is chosen from poly (lactide) (PLLA), polyglycolide (PGA), co-oligomers or copolymers of PLA and PGA (PLGA), poly(anhydrides), poly(trimethylenecarbonates), poly(orthoesters), poly(dioxanones), poly(ε-caprolactones) (PCL), polyesteramide or polythioesteramide.

If R2 is a low molecular weight compound having a Mw<1000 Da, it may be selected from ethane, propane, butane, pentane, ethyleneglycol, diethyleneglycol or triethyleneglycol.

It is clear that the choice of R1, R2 and n in formula II will influence the properties of the resulting polymer, n is preferably chosen from 1-8, more preferably from 2-6. If n=2 a lineair polymer may be formed. If n>2a branched polymer may be formed.

Another object of the present invention is to provide an easy process for the preparation of the thioic acids according to formula I or II.

The process according to the present invention is simplified in that it involves a single step with no side products, hence requiring no or little purification, In addition the process can be easily applied to various functional groups.

The process according to the invention for the preparation of the new thioic acids according to formula I or II comprises the steps of:
a. reacting an alcohol, amine or thiol compound according to formula III
   with glutaric thioanhydride,,succinic thioanhydride or diglycolic-thioanhydride
b. forming a compound of formula I or II

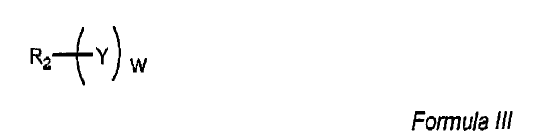

Wherein R2 is a low molecular weight compound having a Mw<1000 Da, oligomer or polymer, Y is OH, NH2, R3NH, SH and w>=1.

w is preferably chosen from 1-8, more preferably from 2-6. R3 may be chosen from the same group as R2. R2 and R3 may be the same or different.

Examples of R2 being a low molecular weight compound having a Mw<1000 Da are ethane, propane, butane, pentane, ethyleneglycol, diethyleneglycol, triethyleneglycol.

Examples of R2 being an oligomer or polymer are PLGA, PLLA, PTMC, PCL and copolymers of PCL and PLA and/or PGA, polyesteramides, polythioesteramides or polyanhydrides.

The process according to the present invention provides the advantage of converting alcohols, amines or thiol functional molecules in one step into a thioic acid functional compound hereby broadening the group of starting molecules that can be converted into thioic acids via an easy route significantly. In the prior art, solely the conversion of carboxylic acid functional molecules to thioic acid functional molecules could be performed in one single step. Moreover, the current route does not require dihydrogensulfide in the conversion of the functional group to a thioic acid group. Dihydrogensulfide is a toxic gas requiring extra safety measures when applied.

It is however also possible to prepare the new thioic acids according to formula I or II by a process comprising the steps of:
a. reacting an alcohol, amine or thiol compound according to formula III with glutaric acid anhydride, succinic acid anhydride or diglycolic acid anhydride forming a compound of formula IV
b. converting compound of formula IV by the use of H₂S to thioic acid(s) according to formula I or II. 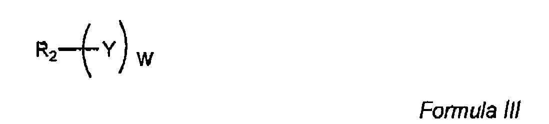
Wherein R2 is a low molecular weight compound having a Mw<1000 Da, oligomer or polymer, Y is OH, NH2, R3NH, SH and w>=1.
w is preferably chosen from 1-8, more preferably from 2-6. R3 may be chosen from the same group as R2. R2 and R3 may be the same or different. 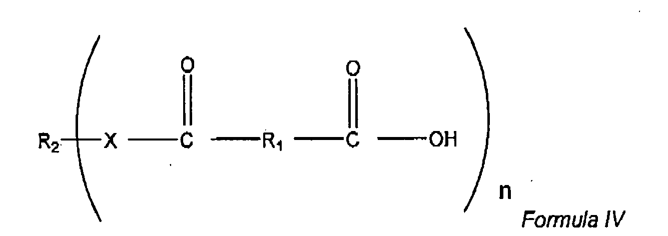 wherein X is O, N or S and n>=1
R1 is chosen from (-CH2-)₂, -CH₂-O-CH₂- or (-CH₂-)₃.

The invention further relates to polythioesters comprising the thioic acids according to formula I and/or II. The type of thioic acids, more specific the ratio of aromatic to aliphatic thioic acids, may influence the polythioester properties, such as degradation or biodegradation, mechanical properties and hydrophilicity and drug solubility and release. When the ratio of aromatic to aliphatic thioic acids increases, the resulting polythioester is likely to be more hydrophobic, and have a higher mechanical strength. Conversely, when the ratio of aromatic to aliphatic thioic acids decreases, the resulting polythioester is likely to be more hydrophilic, and have a lower mechanical strength.

The polythioesters according to the present invention were found to be hydrolysable. Some of them were also found to be biodegradable. Depending on the choice of R2 in formula I or II, being an oligomer or polymer, degradability of the resulting polythioester may be influenced. For instance in case that R2 is a non-degradable triethyleneglycol divinyl ether (TEGDVE), lower degradation rates are achieved when compared to R2 being a degradable polymer such as poly(lactide-co-glycolide)1200di(4-pentenoate) (PLGDP)or poly(lactide-co-glycolide)2600tri(4-pentenoate) (PLGTP). If R2 is chosen being a hydrophobic component such as poly (ε-caprolactone) 2100di (4-pentenoate) (PCLDP) degradation over years is designed.

Particularly suited for applications in the human or animal body, such as in situ applications, is R2 being a polymer that can be degraded into degradation products without leaving any residual components. Such degradation products are then preferably non-toxic. The degradation products may be assimilated in the human or animal metabolism, or excreted.

Polythioesters according to the present invention may be prepared by mixing a compound Z comprising at least one ethylenically unsaturated group with the above described thioic acids of formula I and/or II.

Component Z may be any suitable molecule having a carbon-carbon double bond. For example, the ethylenically unsaturated group may be selected from a group consisting of vinyl, alkyne, alkene, vinyl ether, vinyl sulphones, vinylphosphates, allyl, allylether, allylamine, acrylate, fumarate, maleate, itaconate, crotonate, citraconate, mesaconate, methacrylate, maleimide, isoprene, and norbornene and derivatives thereof such as esters and amides. Cyclic structures may also be used. Preferably component Z is a molecule having an ethylenically unsaturated group selected from a group consisting of alkene, vinyl ether, allyl, acrylate, fumarate, methacrylate, norbornene and derivatives thereof such as esters and amides.

Component Z and the thioic acids of formula I and/or II are reacted by polymerisation. The thioester bonds may be formed employing a wide variety of mechanisms. Light and heat may be suitable or radical initiators but the reaction may also occur spontaneously. The skilled person knows that a wide variety of catalysts, thermal initiators, photoinitiators and stabilizers may be used to influence the speed and extent of the reaction. When used for in situ application in the human or animal body, a blue light or visible light source may be particularly advantageous. Alternatively a spontaneous reaction may be advantageous.

The temperature of the reaction provides a powerful tool to steer the speed and selectivity of the reaction. Also, the concentration of the reactants will determine the speed of the reaction and the properties of the polythioesters obtained.

Marvel and Kotch (J. Amer. Chem. Soc. 73, 1100-1102 (1951) and Kobayashi et al., Polymer Journal, 26, 49 - 59 (1994) and Polymer Journal, 25 507 - 520 (1993) describe a technology wherein components comprising ethylenically unsaturated groups are reacted with components comprising thioic acid groups.

Marvel and Kotch describe the preparation of polythioesters of the type [SCORCOS-R']ₓ from a variety of dibasic chlorides and aliphatic dithiols or from the addition of dibasic thio acids (dithio adipic acid, dithiopimelic acid, dithio suberic acid, dithioazelic acid, dithio sebacic acid, dithio terephtalic acid, dithio isophtalic acid) to the non-conjugated diolefin biallyl (1,5-hexadiene) using W light.

Kobayashi et al., describe the reaction mechanism of the addition reaction of thiobenzoic acid to styrene or ethynylbenzene using UV light or radical initiators (AIBN) in Polymer Journal, 25, 507 - 520 (1993) and the polyaddition of 1,4-benzenedicarbothioic acid to 1,4-divinylbenzene or 1,4-diisopropenylbenzene in Polymer Journal, 26, 49 - 59 (1994). It is to be noted that the starting components used by Marvel and Kotch as well as those used by Kobayashi et al. do not qualify as oligomers or polymers. The starting thioic acids as disclosed in above two citations are hydrophobic thioic acids.

The properties of the polythioesters according to the present invention may further be influenced by the degree of cross-linking. Crosslinking may be achieved by choosing appropriate chain lengths of components Z and of the thioic acids of formula I and/or II. Alternatively, the degree of cross-linking may be influenced by choosing an appropriate number of ethylenically unsaturated groups in component Z. In another alternative the degree of cross-linking may be influenced by preventing the polymerization to go to completion, i.e. by preventing the highest degree of reaction to occur. Preferably, however, the reaction proceeds to the highest degree of reaction. A partial reaction may be especially desirable when it is required to have some residual reactive groups in the cross-linked matrix, for instance for modifications after cross-linking, such as attaching functional groups or covalent attachment to tissue or other biological material.

For the production of particularly useful linear polythioesters it may be advantageous that component Z comprises a maximum of 2 ethylenically unsaturated groups and that the thioic acids of formula I and/or II comprise a maximum of 2 thioic acid groups.

For the production of particularly useful crosslinked polymers or networks, it is required that component Z comprises at least 2 ethylenically unsaturated groups and that the thioic acids of formula I and/or II comprises at least 2 thioic acid groups and that the number of ethylenically unsaturated groups plus thioic acid groups is more than 4.

For the production of particularly strong crosslinked polymers or networks, it is required that component Z comprises at least 3 ethylenically unsaturated groups and/or that the thioic acids of formula I and/or II comprise at least 3 thioic acid groups and that the number of ethylenically unsaturated groµps plus thioic acid groups is more than 5.

For the production of particularly useful crosslinked polymers or networks, it is required that the composition comprising components Z and thioic acids of formula I and/or II fulfils the boundary conditions for compositions for crosslinked polymers or networks as reported by Durand and Bruneau (D. Durand, C.-M. Bruneau, Makromol. Chem. 1982, 183, 1007-1020 and in D. Durand, C.-M. Bruneau, The British Polymer Journal, 1979, 11, 194-198; D. Durand, C.-M. Bruneau, The British Polymer Journal 1981, 13, 33-40; D. Durand, C.-M. Bruneau, Polymer, 1982, 23, 69-72; D. Durand, C.-M. Bruneau, Makromol. Chem., 1982, 183, 1021-1035; D. Durand, C.-M. Bruneau, Polymer, 1983, 24, 587-591).

Components Z and the R2 in the thioic acids of formula I and/or II may be based on the same oligomer or polymer, however, when they are based on different oligomers or polymers, the properties of the resulting polythioesters and distribution of active components such as drugs may be controlled more effectively and the reaction can be steered in a more controllable way. Components Z and the thioic acids of formula I and/or II may both be degradable however one of the components may be partially degradable or non degradable. This is often utilised where an additional property as well as degradability is required.

In the case that degradable polythioesters are of importance component Z and/or the R2 in the thioic acids of formula I and/or II can be both selected from poly (lactide) (PLA), polyglycolide (PGA), co-oligomers or copolymers of PLA and PGA (PLGA), poly(anhydrides), poly(trimethylenecarbonates), poiy(orthoesters), poly(dioxanones), poly(ε-caprolactones) (PCL), poly(urethanes), polyesteramides, polythioesteramides, polyanhydrides, poly (hydroxy acids), polycarbonates, polyaminocarbonates, polyphosphazenes, poly(propylene)fumarates, polyesteramides, polyoxo-esters, poly(maleic acids), polyacetals, polyketals. On the other hand component Z and/or the R2 in the thioic acids of formula I and/or II can also be both selected from natural polymers such as starch, polypeptides, polyhydroxyalkanoates, fibrin, chitin, chitosan, polysaccharides or carbohydrates such as polysucrose, hyaluronic acid, dextran and similar derivatives thereof, heparan sulfate, chondroitin sulfate, heparin, or alginate, and proteins such as gelatin, collagen, albumin, or ovalbumin, or co-oligomers or copolymers, or blends thereof. In a particularly preferred embodiment component Z and the R2 of the thioic acids of formula I and/or II are selected from poly (lactide) (PLA), poly(anhydrides), poly(trimethylenecarbonates, poly(dioxanones), poly(ε-caprolactones) (PCL), poly(lactide-co-glycolide) or co-oligomers or copolymers or blends thereof.

In the case that non degradable polythioesters are required for an additional property like mechanical strength and/or non fouling properties, components Z and/or the R2 of the thioic acids of formula I and/or II may be selected from the group consisting of poly (vinyl alcohol) (PVA), poly (ethylene oxide), poly (ethylene oxide)-co-poly(propylene oxide) block co-oligomers or copolymers (poloxamers, meroxapols), poloxamines, poly(urethanes), poly((polyethyleneoxide)co-poly(butyleneterephtalate)), poly (vinyl pyrrolidone), poly (ethyl oxazoline), carboxymethyl cellulose, hydroxyalkylated celluloses such as hydroxyethyl cellulose and methylhydroxypropyl cellulose. Particularly good non fouling properties were achieved when using poly (ethylene oxide) as component Z and R2 of the thioic acids of formula I and/or II. Particularly good amphiphilic behaviour was achieved when components Z and R2 of the thioic acids of formula I and/or II were selected from the group consisting of poly (ethylene oxide)-co-poly(propylene oxide), poloxamers, poloxamines and meroxapols. Particularly good mechanical strength was achieved when using polyurethanes as polymer in component Z and as R2 of the thioic acids of formula I and/or II. Particularly good hydrophilicity was achieved when components Z and R2 of the thioic acids of formula I and/or II were selected from the group consisting of poly (vinyl pyrrolidone) and poly (ethyl oxazoline).

Multiple components Z and/or thioic acids of formula I and/or II may also be used. Components Z as well as the R2 in the thioic acids of formula I and or II may consist of different oligomers or polymers. Components Z and the thioic acids of formula I and/or II may vary in molecular weight depending upon which properties are desired for the resulting polythioester. More particularly, the molecular weight for component Z and the thioic acids of formula I and/or II may range from about 28 Da to more than about 50000 Da. Prior to formation of the polythioester of the present invention, component Z and the thioic acids of formula I and/or II are derivatized to include thioic acid groups or ethylenically unsaturated groups such that they can participate in thioic-ene polymerisation. For use in *in situ* applications, the component Z and the thioic acids of formula I and/or II are preferably of higher molecular weight.

The polythioesters of the present invention have the advantageous property that they can be degraded hydrolytically or enzymatically. When the components Z and the thioic acids of formula I and/or II are degradable or biodegradable, a polythioester may be synthesized that can be degraded more completely with no residues left.

The invention further relates to medical devices comprising the thioic acids according to formula I or II and/or the polythioesters made thereof. Examples of medical devices are drug eluting balloons, stents, screws, sutures, plates, pins, patches, vascular grafts, tissue engineering scaffoldsor as materials for sutures. The thioic acids according to formula I or II and/or the polythioesters made thereof can however also be used as adhesives, anti-adhesives, sealants, meshes, sponges, gels, waxes, films, cell delivery vehicles, coatings, polymer-based medicinal products or as matrices for the controlled delivery of drugs.The polythioesters are in particular useful as matrix polymer in controlled release systems or drug delivery systems such as in drug-eluting balloons or in other drug-eluting devices.

The invention further relates to microparticles comprising the polythioesters according to the present invention.

As used herein, microparticles include micro- and nanoscale particles which are typically composed of solid or semi-solid materials and which are capable of carrying an active agent. Typically, the average diameter of the microparticles given by the Fraunhofer theory in volume percent ranges from 10 nm to 1000 µm. The preferred average diameter depends on the intended use. For instance, in case the microparticles are intended for use as an injectable drug delivery system, in particular as an intravascular drug delivery system, an average diameter of up to 10 µm, in particular of 1 to 10 µm may be desired.

It is envisaged that nanoparticles typically have an average diameter of less than 1 µm, preferably less than 800 nm, and in particular less than 500 nm. These nanoparticles are very useful for intracellular purposes. For other applications smaller or larger average diameters may be required for instance a diameter in the range of 10-500 nm, 100-300 nm or 500-800 nm. In particular, the particle diameter as used herein is the diameter as determinable by a LST 230 Series Laser Diffraction Particle size analyzer (Beckman Coulter), making use of a UHMW-PE (0.02 - 0.04 µm) as a standard. Particle-size distributions are estimated from Fraunhofer diffraction data and given in volume (%). If the particles are too small or non analyzable by light scattering because of their optical properties then scanning electron microscopy (SEM) or transmission electron microscopy (TEM) can be used.

Several types of microparticle/nanoparticle structures can be prepared. These include substantially homogenous structures. In case that more than one active agent has to be released or in case that one or more functionalities are needed it is preferred that the microparticles/nanoparticles are provided with a structure comprising an inner core and an outer shell. A core/shell structure enables more multiple mode of action for example in drug delivery of incompatible compounds or in imaging. The shell can be applied after formation of the core using a spray drier. The core and the shell may comprise the same or different polymers such as for example the polythioesters according to the present invention, with different active agents. In this case it is possible to release the active agents at different rates. It is also possible that the active agent is only present in the core and that the shell is composed of the polythioesters according to the present invention.

In a further embodiment the microparticles/nanoparticles may comprise a core comprising the polythioesters according to the present invention and a shell comprising another material such as a magnetic or magnetisable material.

In still a further embodiment, the microparticles/nanoparticles may comprise a magnetic or magnetisable core and a shell comprising the polythioesters according to the present invention. Suitable magnetic or magnetisable materials are known in the art. Such microparticles/nanoparticles may be useful for the capability to be attracted by objects comprising metal, in particular steel, for instance an implanted object such as a graft or a stent. Such microparticles/nanoparticles may further be useful for purification or for analytical purposes.

In a still further embodiment, the micro-or nanoparticles may be imageable by a specific technique. Suitable imaging techniques are MRI, CT, X-ray. The imaging agent can be incorporated inside the particles or coupled onto their surface. Such particles may be useful to visualize how the particles migrate, for instance in the blood or in cells. A suitable imaging agent is for example gadolinium.

The microparticles/nanoparticles according to the present invention may carry one or more active agents. The active agent may be more or less homogeneously dispersed within the microparticles/nanoparticles core. The active agent may also be located within the microparticte/nanoparticle shell.

In particular, the active agent may be selected from the group of nutrients, pharmaceuticals, proteins and peptides, vaccines, genetic materials, (such as polynucleotides, oligonucleotides, plasmids, DNA and RNA), diagnostic agents, and imaging agents. The active agent such as an active pharmacologic ingredient (API), may demonstrate any kind of activity, depending on the intended use.

The active agent may be capable of stimulating or suppressing a biological response. The active agent may for example be chosen from growth factors (VEGF, FGF, MCP-1, PIGF, antibiotics (for instance penicillin's such as B-lactams, chloramphenicol), anti-inflammatory compounds, antithrombogenic compounds, anti-claudication drugs, anti-arrhythmic drugs, anti-atherosclerotic drugs, antihistamines, cancer drugs, vascular drugs, ophthalmic drugs, amino acids, vitamins, hormones, neurotransmitters, neurohormones, enzymes, signalling molecules and psychoactive medicaments.

Examples of specific active agents are neurological drugs (amphetamine, methylphenidate), alpha1 adrenoceptor antagonist (prazosin, terazosin, doxazosin, ketenserin, urapidil), alpha2 blockers (arginine, nitroglycerin), hypotensive (clonidine, methyldopa, moxonidine, hydralazine minoxidil), bradykinin, angiotensin receptor blockers (benazepril, captopril, cilazepril, enalapril, fosinopril, lisinopril, perindopril, quinapril, ramipril, trandolapril, zofenopril), angiotensin-1 blockers (candesartan, eprosartan, irbesartan, losartan, telmisartan, valsartan), endopeptidase (omapatrilate), beta2 agonists (acebutolol, atenolol, bisoprolol, celiprolol, esmodol, metoprolol, nebivolol, betaxolol), beta2 blockers (carvedilol, labetalol, oxprenolol, pindolol, propanolol) diuretic actives (chlortalidon, chlorothiazide, epitizide, hydrochlorthiazide, indapamide, amiloride, triamterene), calcium channel blockers (amlodipin, bamidipin, diltiazem, felodipin, isradipin, lacidipin, lercanidipin, nicardipin, nifedipin, nimodipin, nitrendipin, verapamil), anti arthymic active (amiodarone, solatol, diclofenac, enalapril, flecainide) or ciprofloxacin, latanoprost, flucloxacillin, rapamycin and analogues and limus derivatives, paclitaxel, taxol, cyclosporine, heparin, corticosteroids (triamcinolone acetonide, dexamethasone, fluocinolone acetonide), anti-angiogenic (iRNA, VEGF antagonists: bevacizumab, ranibizumab, pegaptanib), growth factor, zinc finger transcription factor, triclosan, insulin, salbutamol, oestrogen, norcantharidin, microlidil analogues, prostaglandins, statins, chondroitinase, diketopiperazines, macrocycli compounds, neuregulins, osteopontin, alkaloids, immuno suppressants, antibodies, avidin, biotin, clonazepam.

The active agent can be delivered for local delivery or as pre or post surgical therapies for the management of pain, osteomyelitis, osteosarcoma, joint infection, macular degeneration, diabetic eye, diabetes mellitus, psoriasis, ulcers, atherosclerosis, claudication, thrombosis viral infection, cancer or in the treatment of hernia.

The fields wherein microparticles/nanoparticles according to the present invention can be used include dermatology, vascular, orthopedics, ophthalmic, spinal, intestinal, pulmonary, nasal, or auricular. Besides in a pharmaceutical application, microparticles/nanoparticles according to the invention may inter alia be used in an agricultural application. In particular, such microparticles/nanoparticles may comprise a pesticide or a plant-nutrient.

The invention will now be illustrated by the following examples without being limited thereto.

### Example 1: Synthesis of thioic acid using succinic anhydrides starting from alcohols

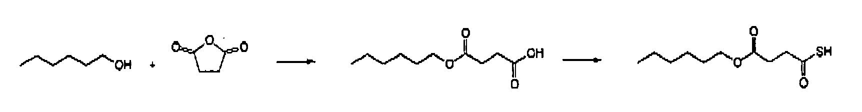

1-Hexanol (1,00 g; 9.8 mmol), pyridine (0.93 g; 11.7 mmol), and DMAP (0.24 g; 1.96 mmol) were dissolved in chloroform (20 mL). Succinic anhydride (1.18 g; 11.7 mmol) was added, and the reaction mixture was stirred at 70°C under an argon atmosphere. After 18hr, the reaction mixture was cooled to 20°C, chloroform (20 mL) was added, and this was washed twice with 1 M hydrochloric acid (20 mL) and water (10 mL). The organic layer was dried with sodium sulfate and evaporated to dryness, to yield the carboxylic acid as a colorless oil (1.64 g; 83%). CDI (0.441 g; 2.72 mmol) was dissolved in chloroform (4 mL) and to this solution was added drop wise a solution of the carboxylic acid (0.50 g; 2.47 mmol) in chloroform (2 mL). Subsequently, the reaction mixture was stirred at room temperature for 1 hr. Then, hydrogen sulfide was bubbled gently through the reaction mixture for 10 min, and the reaction mixture was stirred for an additional 90 min. Diethyl ether (15 mL) was added and the mixture was washed with 0.5 M sulfuric acid (10 mL). The organic layer was dried with sodium sulfate and evaporated to dryness, to yield a yellow oil (0.392 g; 73%).

The product was successfully characterized by ¹H-NMR and FT-IR.
¹H-NMR (CDCl₃): δ= 0.88 (3H), 1.25 (6H), 1.62 (2H), 2.62 (2H), 2.97 (2H), 4.08 (2H) ppm
FT-IR: η=1713, 1731, 2556 cm⁻¹.

### Example 2: Synthesis of thioic acid using glutaric anhydrides starting from alcohols

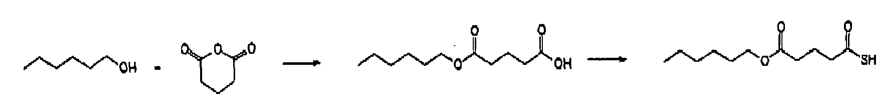

1-Hexanol (1.00 g; 9.8 mmol), pyridine (0.93 g; 11.7 mmol), and DMAP (0.24 g; 1.96 mmol) were dissolved in chloroform (20 mL). Glutaric anhydride (1.14 g; 11.7 mmol) was added, and the reaction mixture was stirred at 70°C under an argon atmosphere. After 18hr, the reaction mixture was cooled to 20°C, chloroform (20 mL) was added, and this was washed twice with 1 M hydrochloric acid (20 mL) and water (10 mL). The organic layer was dried with sodium sulfate and evaporated to dryness, to yield the carboxylic acid as a colorless oil (1.52 g; 72%).

CDI (0.412 g; 2.54 mmol) was dissolved in chloroform (4 mL) and to this solution was added drop wise a solution of the carboxylic acid (0.50 g; 2.31 mmol) in chloroform (2 mL). Subsequently, the reaction mixture was stirred at room temperature for 1hr. Then, hydrogen sulfide was bubbled gently through the reaction mixture for 10 min, and the reaction mixture was stirred for an additional 90 min. Diethyl ether (15 mL) was added and the mixture was washed with 0.5 M sulfuric acid (10 mL). The organic layer was dried with sodium sulfate and evaporated to dryness, to yield yellow oil (0.345 g; 64%).

The product was successfully characterized by ¹H-NMR and FT-IR.
¹H-NMR (CDCl₃): δ = 0.88 (3H), 1.25 (6H), 1.62 (2H), 1.97 (2H), 2.38 (2H), 2.71 (2H), 4.08 (2H), 4.45 (1H) ppm
FT-IR: η= 1713, 1731, 2555 cm⁻¹.

### Example 3: Synthesis of thioic acid using glutaric(thio)anhydride starting from an alcohol

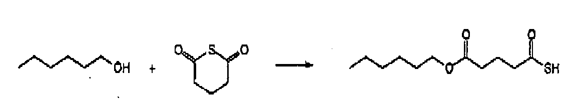

1-Hexanol (100 mg; 0.98 mmol) and DBU (179 mg; 1.17 mmol) were dissolved in chloroform (1.5 mL). Glutaric thioanhydride (127 mg: 0.98 mmol), dissolved in chloroform (1.5 mL) was added, and the reaction mixture was stirred at ambient temperature under an argon atmosphere for 2 hr. Chloroform (10 mL) was added and the mixture was washed with subsequently 0.1 M sulfuric acid (10 mL) and water (10 mL). The organic layer was dried with sodium sulfate and evaporated to dryness, to yield an orange oil (170 mg; 75%). The product was successfully characterized by ¹H-NMR (CDCl₃): δ = 0.88 (t, 3H), 1.30 (m, 6H), 1.60 (m, 2H), 1.95 (m, 2H), 2.35 (t, 2H), 2.68 (t, 2H), 4.05 (t, 2H) ppm.

Within 5 days at 20°C, the product was hardly degraded.

### Example 4: Synthesis of thioic acid using glutaric(thio)anhydrides starting from a primary amine

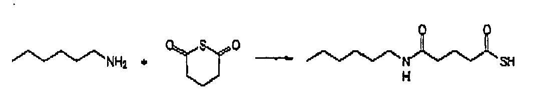

Glutaric thioanhydride (250 mg; 1.92 mmol), hexylamine (233 mg; 2.30 mmol) and triethylamine (233 mg; 2.30 mmol) were dissolved in chloroform (5 mL). The reaction mixture was stirred at ambient temperature under an argon atmosphere for 1 hr. Chloroform (5 mL) was added, and the mixture was washed with subsequently 0.5 M citric acid (10 mL) and water (10 mL). The organic layer was dried with magnesium sulfate and evaporated to dryness, to yield an off white waxy solid (340 mg; 77%). The product was successfully characterized by ¹H-NMR (CDCl₃): δ= 0.88 (t, 3H), 1.30 (m, 6H), 1.47 (m, 2H), 1.98 (m, 2H), 2.22 (t, 2H), 2.72 (t, 2H), 3.22 (q, 2H) ppm.

The product was a little bit degraded during the work-up procedure.

Within 5 days at 20°C, the product was further degraded, mainly to the cyclic imide.

### Example 5: Synthesis of thioic acid using succinic(thio)anhydrides starting from an alcohol

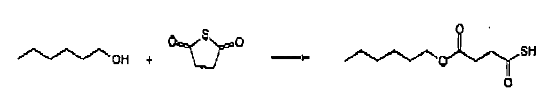

1-Hexanol (200 mg; 1.96 mmol) and Succinic thioanhydride (227 mg: 1.96 mmol) were dissolved in chloroform (4 mL). DBU (358 mg; 2.35 mmol), dissolved in chloroform (2 mL) was added, and the reaction mixture was stirred at ambient temperature under an argon atmosphere for 1 hr. Chloroform (20 mL) was added and the mixture was washed with subsequently 0.1 M sulfuric acid (20 mL) and water (20 mL). The organic layer was dried with sodium sulfate and evaporated to dryness, to yield yellow oil (390 mg: 91%). The product was successfully characterized by ¹H-NMR (CDCl₃): δ = 0.88 (t, 3H), 1.32 (m, 6H), 1.61 (m, 2H), 2.62 (t, 2H), 2.98 (t, 2H). 4.05 (t, 2H) ppm.

The product was hardly degraded during the work-up procedure. Within 7 days at 20°C or -20 °C, the product did not further degradate.

### Example 6: Synthesis of thioic acid using succinic(thio)anhydrides starting from a primary amine

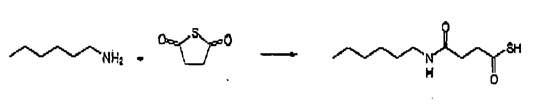

Succinic thioanhydride (250 mg; 2.15 mmol), hexylamine (261 mg; 2.58 mmol) and triethylamine (261 mg; 2.58 mmol) were dissolved in chloroform (6 mL). The reaction mixture was stirred at ambient temperature under an argon atmosphere for 30 min. Chloroform (5 mL) was added, and the mixture was washed with subsequently 0.5 M citric acid (10 mL) and water (10 mL). The organic layer was dried with sodium sulfate and evaporated to dryness, to yield a white solid (370 mg; 79%). The product was successfully characterized by ¹H-NMR (CDCl₃): δ = 0.88 (t, 3H), 1.32 (m, 6H), 1.48 (m, 2H), 2.46 (t, 2H), 3.02 (t, 2H), 3.22 (q, 2H) ppm.

The product was slightly degraded during the work-up procedure. Within 7 days at -20 °C, the product did not further degradate. Within 7 days at 20 °C, the product was degraded for more than 50%, mainly to the cyclic imide.

### Example 7: Synthesis of thioic acid using glutaric (thio)anhydrides starting from a secondary amine

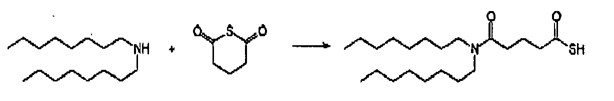

Glutaric thioanhydride (250 mg; 1.92 mmol), N,N-dioctylamine (464 mg; 1.92 mmol), and triethylamine (194 mg; 1.92 mmol) were dissolved in chloroform (4 mL). The reaction mixture was stirred at 20°C under an argon atmosphere. After 45 min, chloroform (6 mL) was added, and this was washed four times with 0.1 M hydrochloric acid (8 mL). The organic layer was dried with sodium sulfate and evaporated to dryness, to yield a waxy solid (470 mg; 66%).

The product was successfully characterized by ¹H-NMR.

Within 6 days at 20°C, the product was hardly degraded.
¹H-NMR (CDCl₃): δ = 0.88 (6H). 1.25 (20H), 1.50 (4H), 1.98 (2H), 2.38 (2H), 2.75 (2H), 3.18 (2H), 3.25 (2H) ppm

### Example 8: Synthesis of thioic acid using succinic (thio)anhydrides starting from a thiol

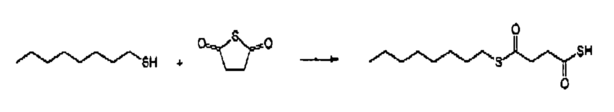

Succinic thioanhydride (58 mg; 0.50 mmol), 1-octanethiol (73 mg: 0.50 mmol), and triethylamine (51 mg; 0.50 mmol) were dissolved in chloroform (1 mL). The reaction mixture was stirred at 20°C under an argon atmosphere. After 1hr, chloroform (3 mL) was added, and this was washed twice with 0.1 M hydrochloric acid (2.5 mL). The organic layer was dried with sodium sulfate and evaporated to dryness, to yield a yellow oil (84 mg; 64%).

The product was successfully characterized by ¹H-NMR.

Within 4 days at 20°C, the product was degraded significantly.
¹H-NMR (COCl₃): □ 0.88 (3H), 1.25 (10H), 1.58 (2H), 2.88 (4H), 2.99 (2H) ppm

### Example 8: Synthesis of thioic acid using glutaric (thio)anhydrides starting from a thiol

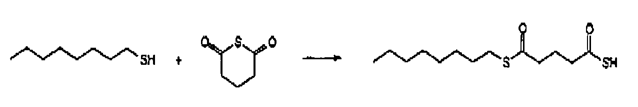

Glutaric thioanhydride (250 mg; 1.92 mmol), 1-octanethiol (280 mg; 1.92 mmol), and triethylamine (194 mg; 1.92 mmol) were dissolved in chloroform (4 mL). The reaction mixture was stirred at 20°C under an argon atmosphere. After 1hr, chloroform (6 mL) was added, and this was washed twice with 0.1 M hydrochloric acid (10 mL). The organic layer was dried with sodium sulfate and evaporated to dryness, to yield yellow oil (458 mg; 86%).

The product was successfully characterized by ¹H-NMR.

Within 4 days at 20°C, the product was hardly degraded.
¹H-NMR (CDCl₃): □ 0.88 (3H), 1.25 (10H), 1.57 (2H), 2.00 (2H), 2.61 (2H), 2.70 (2H), 2.87 (2H), 4.66 (1H) ppm.

### Example 9: Synthesis of thioic acid using glutaric (thio)anhydrides starting from polyethyleneglycol (PEG).

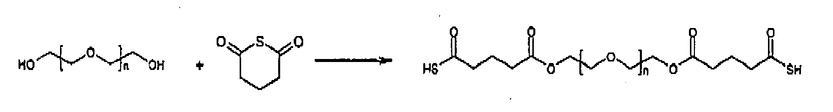

Poly ethyleneglycol (Mw=1000; 22.93 g; 22.93 mmol) and glutaric thioanhydride (5.97 g; 45.86 mmol) were dissolved in chloroform (200 mL). DBU (8.38 g; 55.04 mmol), dissolved in chloroform (50 mL) was added, and the reaction mixture was stirred at ambient temperature under an argon atmosphere for 3 hr. Chloroform (250 mL) was added, and the mixture was washed with subsequently 0.1 M sulfuric acid (400 mL) and water (400 mL). The organic layer was dried with sodium sulfate and evaporated to dryness, to yield a brownish oil. The oil was co-evaporated twice with diethyl ether yielding a yellow waxy solid (27.2 g: 94%). The product was successfully characterized by ¹H-NMR (CDCl₃): □ 1.95 (m, 4H), 2.41 (1, 4H), 2.73 (t, 4H), 3.61 (m, 88H), 3.68 (t, 4H), 4.22 (t, 4H) ppm, ¹³C-NMR (CDCl₃): δ 20.28, 32.71, 44.62, 63.63, 69.07, 70.37, 70.58, 70.62, 172.61, 197.02 ppm and FT-IR: ν = 2867, 1732 (C=O stretch), 1699 (C=O stretch) 1453, 1349, 1297, 1249, 1094 (C-O stretch), 1037, 948, 846 cm⁻¹.

### Example 10: Linear polymers based on PEG-1000 dithioic acid and PLGA.

5.75 µL of dimethylethanolamine (DMEA) and 1.27 g of acetone were added to 0.9998 g (0.189 mmol) of a poly(D,L-lactide-co-glycolide) (Mₙ=5282 Da)dipentenoate with a lactide to glycolide ratio of 75:25. The mixture was allowed to mix overnight in the dark at room temperature by shaking (140 movements/min). Subsequently, 0.1615 g (0.0189 mmol) of a PEG-1000 dithioic acid (Mₙ=855 Da) was added. The mixture was shaken under the same conditions as mentioned above for 2 h. Finally, 0.015 mL of photoinitiator (Darocur 1173, 1 wt% w/w) was added and the mixture was shaken for an additional 30 minutes. The composition was applied onto a glass plate using a doctor blade (height=200 µm). The polymerization was performed using UV-light (Fusion F600 D-bulb, λ=320-380 nm, dose 15.2 J/cm²). The obtained material was placed in the oven at a temperature of 35 °C under vacuum overnight to remove any remaining volatile components to yield an off-white solid, which did not dissolve in water.

## Claims

1. Thioic acids according to formula I; 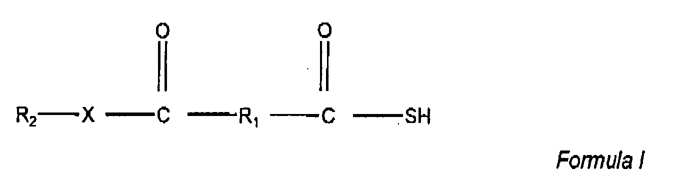 Wherein X is O, N or S and whereby
R1 is chosen from (-CH₂-)₂, -CH₂-O-CH₂- or (-CH₂-)₃
R2 is chosen from a low molecular weight compound having a Mw<1000 Da, an oligomer or a polymer with the proviso that
if R1=(-CH2-)₂ and X=O, R2 is chosen from a non-degradable polymer with a Mw>6000, a degradable polymer or oligomer, an aliphatic compound comprising at least 2 C atoms and a Mw<5000 or an aromatic compound comprising at least 2 aromatic rings;

2. Thioic acids according to claim 1 whereby R1 = (-CH2-)₂ and R2 is chosen from polyethyleneglycol (PEG) with a Mw>6000 or a degradable polymer or oligomer.

3. Thioic acids according to claim 1 whereby R1 = (-CH2-)₃ and R2 is chosen from a degradable or non-degradable monomer, oligomer or polymer.

4. Thioic acids according to any one of the claims 1-3 whereby R2 is a degradable oligomer or polymer chosen from poly (lactide) (PLLA), polyglycolide (PGA), co-oligomers or copolymers of PLA and PGA (PLGA), poly(anhydrides), poly(trimethylenecarbonates), poly(orthoesters), poly(dioxanones), poly(ε-caprolactones) (PCL), polyesteramides.

5. Thioic acids according to formula II; 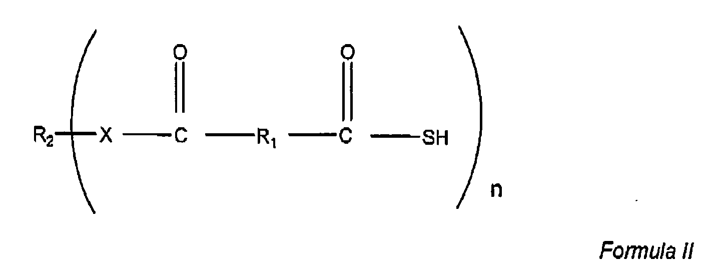 Wherein X is O, N or S and n >1;
R1 is chosen from (-CH₂-)₂, -CH₂-O-CH₂- or (-CH₂-)₃,
R2 is chosen from a low molecular weight compound having a Mw<1000 Da, an oligomer or a polymer.

6. Thioic acids according to claim 5 whereby R2 is chosen from a degradable oligomer or polymer.

7. Thioic acids according to claim 6 whereby R2 is chosen from a degradable oligomer or polymer chosen from poly (lactide) (PLLA), polyglycolide (PGA), co-oligomers or copolymers of PLA and PGA (PLGA), poly(anhydrides), poly(trimethylenecarbonates), poly(orthoesters), poly(dioxanones), poly(ε-caprolactones) (PCL), polyesteramides.

8. Thioic acids according to any one of the claims 5-7 whereby n=2.

9. Thioic adds according to any one of the claims 5-7 whereby n>2.

10. Process for the preparation of the thioic acids according to any one of the claims 1-9 comprising the steps of
a. reacting an alcohol, amine or thiol compound according to formula III with glutaric thioanhydride, succinic thioanhydride or diglycolic thioanhydride
b. forming a compound of formula I or II 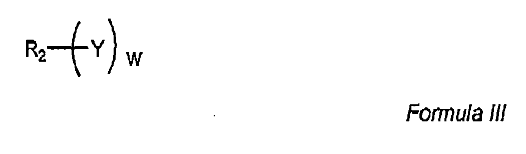
Wherein R2 is a low molecular weight compound having a Mw<1000 Da, oligomer or polymer, Y is OH, NH2, R3NH, SH and w>=1. R3 may be chosen from the same group as R2. R2 and R3 may be the same or different.

11. Process for the preparation of the thioic acids according to any one of the claims 1-9 said process comprising the steps of
a. reacting an alcohol, amine or thiol compound according to formula III with glutaric acid, succinic anhydride, diglycolic anhydride forming a compound of formula IV
b. converting compound of formula IV by the use of H₂S to thioic acids according to formula I or II, 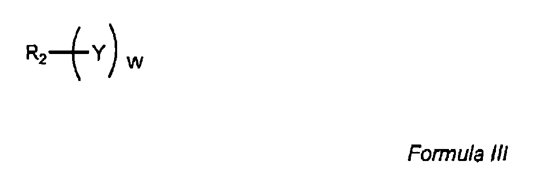 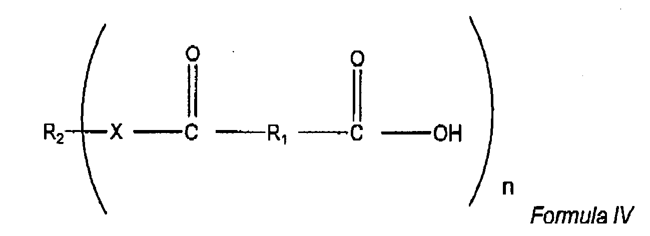
wherein X is chosen from O, N or S and w>=1, R1 is chosen from (-CH₂-)₂, -CH₂-O-CH₂- or (-CH₂-)₃
R2 is a low molecular weight compound having a Mw<1000 Da, oligomer or polymer,
Y is OH, NH2, R3NH, SH and wherein R3 may be chosen from the same group as R2. R2 and R3 may be the same or different.

12. Polythioesters prepared by using the thioic acids according to any one of the claims 1-9.

13. Polythioesters according to claim 12 prepared by mixing a compound Z comprising at least one ethylenically unsaturated group with the thioic acids according to formula I and/or II.

14. Polythioesters according to claim 13 wherein the ethylenically unsaturated group of compound Z is chosen from alkene, vinyl ether, allyl, acrylate, fumarate, methacrylate or norbornene.

15. Medical device comprising the polythioesters according to any one of the claims 12-14.

16. Use of the polythioesters according to any one of the claims 12-14 or the medical devices according to claim 15 in drug delivery applications.

17. Micro-or nanoparticles comprising the thioic acids according to any one of claims 1-9 or polythioesters according to any one of the claims 12-14.
